(19) Europäisches Patentamt
European Patent Office
Office européen des brevets

(11) EP 2 167 056 B1

(12) **EUROPEAN PATENT SPECIFICATION**

(45) Date of publication and mention of the grant of the patent:
**01.05.2019 Bulletin 2019/18**

(21) Application number: **08750563.2**

(22) Date of filing: **09.05.2008**

(51) Int Cl.:
***A61K 9/70*** (2006.01) ***C07D 265/30*** (2006.01)

(86) International application number:
**PCT/GB2008/001627**

(87) International publication number:
**WO 2008/139170 (20.11.2008 Gazette 2008/47)**

(54) **TRANSDERMAL AND TRANSMUCOSAL DRUG DELIVERY ENHANCERS**

TRANSDERMALE UND TRANSMUKOSALE ARZNEIABGABE-VERSTÄRKER

AGENTS AMÉLIORANT L'ADMINISTRATION TRANSDERMIQUE ET TRANSMUCOSALE DE MÉDICAMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.05.2007 GB 0708960**

(43) Date of publication of application:
**31.03.2010 Bulletin 2010/13**

(73) Proprietor: **Merial, Inc.**
**Duluth, GA 30096 (US)**

(72) Inventors:
- **CHAMBER, Ola**
  **Surrey GU7 1XW (GB)**
- **THELIN, Brent**
  **Surrey GU7 1XW (GB)**

(74) Representative: **D Young & Co LLP**
**Briton House**
**Briton Street**
**Southampton SO14 3EB (GB)**

(56) References cited:
**EP-A- 0 268 222** **WO-A-98/11222**
**WO-A-2007/010294**

- **MEALY N ET AL: "Delmopinol hydrochloride. Dental plaque formation inhibitor, treatment of gingivitis" DRUGS OF THE FUTURE, PROUS SCIENCE, ES, vol. 21, no. 8, 1 January 1996 (1996-01-01), pages 787-791, XP009080745 ISSN: 0377-8282**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to the field of drug delivery systems and more specifically to the use of vehicle substances for enhancing the penetration of pharmacologically active agents through human and animal skin and mucosa.

### Background to the Invention

It is known that intact skin can be the site of drug administration to provide continuous, controlled drug delivery into the systemic circulation and there are a number of therapeutically active agents which are best applied topically to obtain the desirable results. This so-called "transdermal" drug delivery offers several potential advantages, including the avoidance of gastrointestinal incompatibility with the drugs and the avoidance of unwanted destruction of the drugs by metabolism in the gastrointestinal tract or by "first pass" hepatic metabolism. Transdermal absorption has been found to provide a constant drug concentration in the body and to offer improved efficiency, i.e. an improved therapeutic effect with minimal side effects.
Continuous drug administration permits the use of drugs with a short biological half-life. An efficient transdermal administration system can be compared with very slow intravenous therapy. Transdermal administration can therefore be considered to provide most of the advantages of intravenous dosing without the risks and inconveniences of intravenous therapy.
When using skin as a route for drug administration, the drug must have suitable physico-chemical properties for efficient transdermal permeation, so that a therapeutically active concentration in the body can be reached and maintained for a desired period. Most drugs, however, have suboptimal characteristics in this respect and can not simply be administered topically. Attempts to achieve full control of the percutaneous absorption give rise to major problems not only because of the relative impermeability of human skin, but also because of its considerably large biological variability.
One approach to render the skin more permeable for a therapeutically active drug is to administer the drug in combination with an absorption promoting compound, often referred to as a penetration enhancer.
It is evident that nasal administration is a useful way for the delivery of drugs to the systemic circulation. Recently, the aim of using the nasal route was for local action on the mucosa. However, several advantages can be achieved by using this route for administering drugs to the systemic circulation, such as the large surface area of the nasal cavity, the highly vascularised, highly permeable nature of the mucosa and its low metabolizing capacity.
The most pronounced drawback with nasal administration is the relatively low bioavailability of the administered drug, which is very much dependent on the molecular size of the drug. Accordingly, systems which promote mucosal permeability would be useful. Absorption enhancers such as bile acids and surfactants have been used for this purpose. However, they are associated with toxic effects on the nasal mucosa. Therefore, there is a need for other kinds of substances which enhance the penetration of active substances through the nasal mucosa.
Various compounds have been tested as penetration enhancers and they can be divided into three groups: solvents, surfactants and miscellaneous.
Solvents suitable for promoting enhancement of drugs include water, alcohols, alkyl methyl sulfoxides, especially dimethyl sulfoxide (DMSO), pyrrolidones and laurocapram (1-dodecylazacycloheptan-2-one; Azone). The mechanisms underlying the enhancing effects of solvents are still not fully understood.
Surfactants can be anionic (e.g. sodium dodecyl sulfate), cationic (e.g. cetyltrimethyl ammonium bromide), or nonionic, in which case the polar group is normally a polyoxyethylene chain (e.g. polyoxyethylene sorbitan monopalmitate). Although surfactants have been tested as penetration enhancers, a number of surfactants are irritating to the skin and/or mucosa and for this reason have been of limited interest.
Into the miscellaneous group may be classified urea, N,N-diethyl-m-toluamide, calcium thioglycolate, anticholinergic agents such as benzilonium bromide and oxyphenonium bromide, and saturated as well as unsaturated fatty acid alcohol esters. Fatty acids and alcohols and esters thereof can also be used as penetration enhancers.
Often, elements from the groups discussed above are combined. The enhancer formulation is in this case designed to contain a solvent for rapid onset as well as a fatty/non-polar component for drug-release over a sustained period.
The state-of-the-art as regards enhancement promoting compounds is described in Kenneth A. Walters "Penetration enhancers and their use in Transdermal Therapeutic Systems" in Hadgraft & Guy "Transdermal Drug Delivery", p. 197 - 229, Marcel Dekker Inc., New York and Basel, 1989.
Systems of the types discussed above have been found to work acceptably in certain cases and especially in connection with specific drugs, but there is clearly a need for improvements in means and methods for administration

of drugs under conditions to give therapeutically efficient results with no or substantially no irritating or allergic side effects.

Summary of the Invention

The present invention provides new and efficient means and methods for the enhanced administration of drugs without the above-mentioned side effects.

According to a first aspect of the invention, a composition comprises a compound of Formula (I) or a pharmacologically acceptable salt thereof and a pharmacologically active agent:

(I)

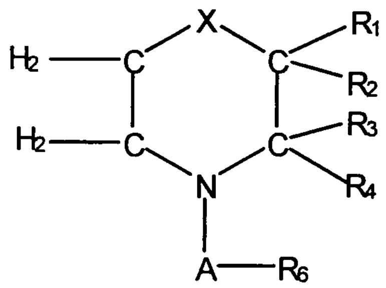

wherein

X is O or CH - $R_5$
$R_5$ is H or branched or straight $C_5$-$C_{14}$ alkyl,
especially 2-propylpentyl; and
when X is O then
$R_1$, $R_2$, $R_3$ and $R_4$ each independently is H or at most two of them being branched or straight $C_4$- $C_{16}$ alkyl, the remaining being H,
A is $(CH_2)_{2-6}$
$R_6$ is OH;
and
when X is CH - $R_5$ then
$R_1$, $R_2$, $R_3$ and $R_4$ is H
A is $(CH)_{2-6}$ or $CO(CH_2)_{6-14}$
$R_6$ is $CH_3$ or OH;
and N in formula (I) optionally being quaternized,
including racemic and enantiomeric forms,

According to the second aspect of the invention, a compound of Formula (I) is used in therapy.

According to a third aspect of the invention, a compound of Formula (I) is suitable for nasal administration.

According to a fourth aspect of the invention, a transdermal or transmucosal penetration enhancing composition comprises a compound of Formula (I).

A fifth aspect of the invention comprises a method of delivering a pharmacologically active agent across a dermal or mucosal barrier, comprising administering a compound of Formula (I) to the barrier, sequentially or simultaneously to administering the pharmacologically active agent.

According to a sixth aspect of the invention, a method of transdermal or transmucosal drug delivery comprises contacting the skin or mucosa of a subject with a compound of Formula (I), sequentially or simultaneously to contact with a pharmacologically active agent.

According to the seventh aspect of the invention, a composition comprises a compound of Formula (I) as a transdermal or transmucosal drug-delivery vehicle.

According to an eighth aspect of the invention, a compound of Formula (I) is used as a transdermal or transmucosal delivery agent.

According to a ninth aspect of the invention, a method of increasing transdermal or transmucosal drug delivery comprises administering, together with a drug, a compound of Formula (I) to the skin or mucosa of a Patient.

According to the tenth aspect of the invention, a woven material comprises a composition according to the first aspect.

According to the eleventh aspect of the invention, a container for nasal administration comprises a composition according to the first aspect.

According to the twelfth aspect of the invention, an aerosol container comprises a composition according to the first aspect.

A thirteenth aspect of the invention provides a novel compound having the formula 4-(2-hydroxyethyl)-4-methyl-3-(4-propylheptyl)morpholinium chloride, i.e.

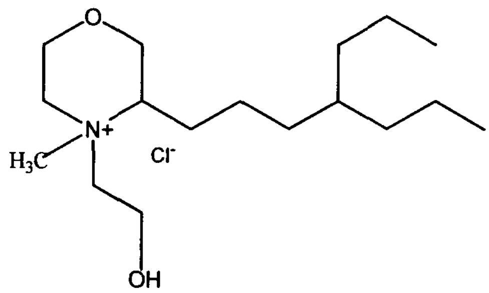

Detailed Description of the Figure

The invention is described with reference to the accompanying drawing wherein Figure 1 illustrates the reduction in plasma glucose after administration of insulin with and without octapinol hydrochloride.

Detailed Description of the Invention

Compounds of Formula (I) are effective as penetration enhancers, to increase absorption through the skin and/or mucosa. The term "skin" is to be given its normal meaning in the art, i.e. the external organ comprising epithelial tissue. The term "mucosa" is well known in the art and comprises all mucous barriers in the body, including the gastro-intestinal, pulmonary (including bronchial), oral (including sublingual and buccal), rectal, vaginal, nasal, urethral, oesophageal and ocular barriers. The skilled person will realise that penetration through skin is "transdermal" and penetration through mucosa is "transmucosal", Compounds of Formula (I) are effective transdermal and transmucosal penetration enhancers and compounds of Formula (I) can therefore be considered as transdermal or transmucosal delivery vehicles, or delivery agents.

A transdermal or transmucosal penetration enhancing composition comprises a compound of Formula (I). The compounds of Formula (I) are effective as penetration enhancers on both human and animal skin and mucosa.

The transdermal or transmucosal penetration of a pharmacologically active agent is enhanced by the presence of a compound of Formula (I). As used herein, the term "pharmacologically active agent" refers to an organic or inorganic, natural or synthetic substance that changes one or more functions of a living organism. The pharmacologically active agent is preferably therapeutic. A common alternative name for a pharmacologically active agent is a "drug", which is to be given its usual meaning in the art. The transmucosal or transdermal penetration is "enhanced". This refers to an increase in the penetration of the pharmacologically active agent through the skin or mucosa. For example, the penetration of an agent that can pass through the skin or mucosa unaided can be improved using a compound of Formula (I). Alternatively, an agent that would not otherwise penetrate the skin or mucosa can penetrate when combined with a compound of Formula (I).

The compounds of Formula (I) are, in one embodiment, used to deliver a pharmacologically active agent for local action, so that the pharmacologically active agent has its effect in the area of contact between the agent and skin or mucosa. For example, a locally active agent, when applied to the nasal mucosa, will have its pharmacological effect on the nasal mucosa. In a separate embodiment, the compounds of Formula (I) are used to deliver the pharmacologically active agent for systemic action, affecting the body as a whole rather than a localised, isolated area. A preferred embodiment is delivery of a pharmacologically active agent to the nasal mucosa, for systemic effect. The terms "systemic" and "localised" are known in the art and are to be given their usual meaning.

The invention relates to a composition comprising a compound of Formula (I) and a pharmacologically active agent. The compound of Formula (I) enhances the transdermal or transmucosal penetration of the pharmacologically active agent. The compound of Formula (I) therefore acts as a vehicle that facilitates the passage of the pharmacologically active agent across the skin and/or mucosa. Preferably, the compound of Formula (I) is present exclusively as a penetration-enhancing vehicle and does not itself have a pharmacological effect, more preferably it does not have a therapeutic pharmacological effect. For example, in a preferred embodiment wherein a composition comprises a compound of Formula (I) and an analgesic, the composition will be used to treat pain. The analgesic is therefore the only active pharmacological agent in the composition, as the compound of Formula (I) does not have analgesic properties.

A composition according to the invention is useful in therapy. As detailed above, the therapeutic utility is due

to the presence of the pharmacologically active agent and not the compound of Formula (I). However, the presence of the compound of Formula (I) enhances the therapeutic utility of the pharmacologically active agent when it is delivered transmucosally or transdermally. This surprising synergy could not have been predicted.

For the avoidance of doubt, a composition comprising a pharmacologically active agent and a compound of Formula (I) is preferably used in therapy wherein the therapy is the prevention or treatment of a condition against which the pharmacologically active agent is effective but the compound of Formula (I) is not. The term "condition" includes any disease, disorder, illness, dysfunction or undesirable affliction that can be prevented or treated using a pharmacologically active agent. Preferred conditions include pain, impotence and drug dependence, in particular nicotine dependence. When the condition to be treated or prevented is pain, the pharmacologically active agent is preferably an analgesic. When the condition to be treated or prevented is impotence, the pharmacologically active agent is preferably sildenafil, tadalafil or vardenafil. When the condition to be treated or prevented is nicotine dependence, the pharmacologically active agent is preferably nicotinic acid.

A composition according to the invention is intended for transmucosal or transdermal delivery. The composition will therefore be brought into contact with a dermal or mucosal surface. The dermal or mucosal surface can be on or in the human or animal body, as detailed above. Preferably, the surface is human mucosa or skin. A preferred surface is the nasal mucosal surface.

A composition according to the invention will be prepared in a formulation suitable for transdermal and/or transmucosal delivery. Suitable formulations will be apparent to the skilled person and will depend on the precise location to which the composition is to be delivered. For example, a microparticulate composition suitable for inhalation, for delivery to the nasal, oesophageal or pulmonary mucosa, is a preferred formulation. An inhalable composition, for example comprising a microparticle dry powder, aerosol formulation or fine spray, will also be suitable for nasal administration of a composition according to the invention. Containers are known in the art that are designed to deliver drugs to the nasal mucosa; these generally comprise a slender nozzle for insertion into the nostril and means for delivering the drug formulation from the container to the nasal mucosa. Therefore, in a preferred embodiment, a composition according to the invention is provided in a container intended for nasal administration of the composition. In an alternative preferred embodiment, a composition according to the invention is prepared as an aerosol formulation, preferably in an aerosol container, yet more preferably an aerosol canister. Aerosol canisters are known in the art and generally comprise a container, a valve and a valve actuator or button.

A composition comprising a compound of Formula (I) can be applied to the skin or mucosa in any suitable formulation. Preferred formulations include a cream, ointment, salve, lotion, gel, or paste.

The formulation can be provided in any suitable packaging or a device; example of suitable packaging includes a tube (preferably similar to a toothpaste tube), a pot or tub, a canister or similar. In a preferred embodiment, the packaging is sterile, yet more preferably for single use only. An example of a suitably packaged formulation is a cream, gel or salve packaged in a tube, preferably a tube of the type used to package toothpaste.

In a further preferred embodiment, a woven material comprises a composition according to the invention, in any formulation. The woven material is preferably a textile, such as a bandage, yet more preferably in the form of a wound dressing, yet more preferably a sterile (aseptic) wound dressing. In yet a further preferred embodiment, the woven material is adhesive or has an adhesive layer, preferably self-adhesive. An example of a suitable adhesive woven material comprising a composition according to the invention is an adhesive bandage, commonly referred to as a "plaster" or "sticking plaster".

Adhesive materials, preferably adhesive woven materials, for the delivery of a pharmacologically active agent to the skin are known in the art as a "transdermal patch" or "skin patch". These are most commonly used for delivery of nicotinic acid. Transdermal (and transmucosal) patches comprising a composition according to the invention are within the scope of the present invention. Such patches often contain multiple layers, including a membrane and an adhesive layer.

A composition comprising a compound of Formula (I) and a pharmacologically active agent is applied to the skin or mucosa of a human or animal. This application is preferably topical, i.e. only a small, restricted area of the skin or mucosa is contacted with the composition; for the avoidance of doubt the term "topical" is to be given its usual meaning in the art.

A method of delivering a pharmacologically active agent, i.e. a drug, across a dermal or mucosal barrier requires the administration of a compound of Formula (I) to a dermal or mucosal barrier of a human or animal subject, sequentially or simultaneously to administration of the pharmacologically active agent to the dermal or mucosal barrier. Preferably, the compound of Formula (I) is administered simultaneously with administration of the pharmacologically active agent. However, it is possible to administer the compound Formula (I) before or after the pharmacologically active agent and obtain the penetration-enhancing benefits. If the compound of Formula (I) and pharmacologically active agent are not applied simultaneously, then it is preferred the compound of Formula (I) is applied to the skin or mucosa before the pharmacologically active agent.

Once the compound of Formula (I) and pharmacologically active agent have both contacted the skin or mucosa,

the action of the compound of Formula (I) will enhance the transdermal or transmucosal penetration of the pharmacologically active agent, which will preferably have its pharmacological effect once the skin or mucosa has been penetrated.

A few compounds of special interest to be used according to the present invention are illustrated below.

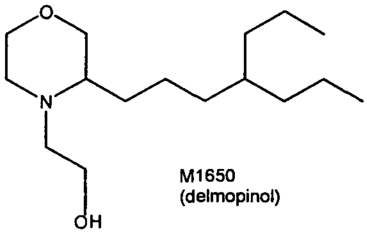

M1650
(delmopinol)

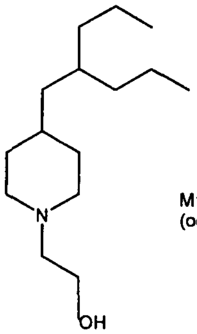

M1523
(octapinol)

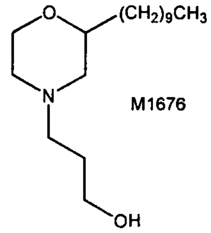

M1676

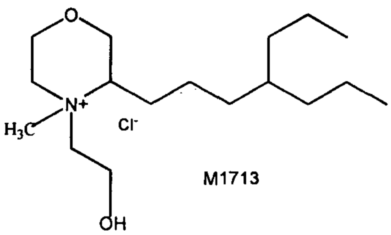

M1713

These substances, except M1713, and related analogues of potential importance in methods according to this invention are disclosed in US patents 4,235,875, 4,894,221 and 4,636,382, which are hereby incorporated by reference. Methods for preparation of delmopinol as well as analogues, except M1713, of the substance are disclosed in said patents and also in PCT application WO 90/1 4342.

In a preferred embodiment, octapinol (M1523) and/or delmopinol (M1650) is used in a composition to enhance transdermal or transmucosal penetration of a pharmacologically active agent, yet more preferably across the nasal mucosa and yet more preferably wherein the active agent is a peptide.

The compound M1713, i.e. 4-(2-hydroxyethyl)-4-methyl-3-(4-propylheptyl)-morpholinium chloride, was prepared in the following way: 10 g of delmopinol (M1650) and 15 g of methyl iodide in 100 ml of ethanol (99%) was refluxed for 2,5 hrs. The reaction mixture was then evaporated in vacuo whereupon 1 l of diethyl ether was added. The mixture was placed in -20°C over night. The solvent was decanted and the residue dissolved in 250 ml of destilled water. This solution was eluted through a column with Amberlite IRA-400 ($OH^-$). The eluate, approx. 1 l, was acidified with hydrochloric acid and evaporated in vacuo at 50°C. 500 ml of ethanol (99%) was added and the solvent was again evaporated. The crystal residue was dried in vacuo and recrystallized from acetone. The yield was 2.5 g (m.p. 140-141°C).

| | | | | | |
|---|---|---|---|---|---|
| $C_{17}H_{36}C1\ NO_2$ (321.9) | calc. | C 63.42 | H 11.27 | N 4.35 | $Cl^-$ 11.01 |
| | found | 62.5 | 11.45 | 4.36 | 11.2 |

$^1$H-NMR (CDCl$^3$): δ 0.9 (6H, $CH_3$), 1.2-1.4 (15H, $CH_2$), 1.9 (1H.OH), 3.4 (3H,$CH_3$-N+), 3.7-4.3 (11H, CH-N+, $CH_2$-N+, $CH_2$-O)

$^{13}$C-NMR (CDCl$^3$): δ 14.7 ($CH_3$), 19.8 ($CH_2$), 23.8 ($CH_2$), 25.4 ($CH_2$), 33.8 ($CH_2$), 35.8 ($CH_2$), 36.9 (CH), 42.7 ($CH_3$-N+), 55.5 ($CH_2$-OH), 60.8 ($CH_2$-N+), 61.1 ($CH_2$-N+), 64.4 ($CH_2$-O), 66.5 ($CH_2$-O), 69.6 (CH-N+)

The compounds of Formula (I) can be used in their free base form or as a pharmaceutically acceptable salt thereof. Examples of pharmaceutically acceptable salts are the salts of acids such as acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid and the like. The most preferred salts are those of hydrochloric acid. An example of a suitable salt is delmopinol hydrochloride (CAS No. 98092-92-3).

One or more pharmacologically active agents are combined with one or more compounds of Formula (I) to provide a composition according to the invention. The one or more pharmacologically active agents are present in the composition at a therapeutically effective level, which will differ for each agent but will be readily apparent to the skilled person. The one or more compounds of Formula (I) will be present at a level sufficient to enhance the transdermal or

transmucosal penetration of the pharmacologically active agent or agents. The compound of Formula (I) is preferably present at greater than 0.01% w/v, more preferably greater than 0.02% w/v, yet more preferably greater than 0.2% w/v, for example 1% w/v or more, 2% w/v or more, 5% w/v or more, 10% w/v or more, 15% w/v or more. The preferred maximum amount of the compound of Formula (I) in the composition is 25% w/v. A preferred range is between 0.1% w/v and 2% w/v.

The pharmacologically active agent or agents included in the delivery system of the invention may be selected from many therapeutic groups including steroidal and non-steroidal anti-inflammatory drugs (e.g. ibuprofen, indomethacin, naproxen, diclofenac, tolfenamic acid, piroxicam), analgesics (e.g. buprenorphine, codeine, fentanyl, morphine, hydromorphone), anti-impotence (e.g. sildenafil, tadalafil or vardenafil), tranquilizers (e.g. diazepam, droperidol, fluspirilene, haloperidol, lorazepam, propiomazin), cardiac glycosides (e.g. digoxin, ouabain), narcotic antagonists (e.g. naloxone, nalorphine), antiparkinsonism agents (e.g. bromocriptine, biperiden, benzhexol, benztropine), antidepressants (e.g. imipramine, nortriptyline, protriptylene), antineoplastic agents and immunosuppressants (e.g. bleomycin, cyclosporin A, fluorouracil, mercaptopurine, methotrexate, mitomycin), antiviral agents (e.g. idoxuridine, acyclovir, interferons, vidarabin), antibiotic agents (e.g. clindamycin, erythromycin, fusidic acid, gentamicin), appetite suppressants (e.g. fenfluramine, mazindol, phentermin), antiemetics (e.g. metoclopramide, droperidol, haloperidol, promethazine), antihistamines (e.g. chlorpheniramine, terfenadine, triprolidine), antimigraine agents (e.g. dihydroergotamine, ergotamine, pizotyline), coronary, cerebral or peripheral vasodilators (e.g. nifedipine, diltiazem), antianginals (e.g. glyceryl trinitrate, isosorbide dinitrate, molsidomine, verapamil), calcium channel blockers (e.g. verapamil, nifedipine, diltiazem, nicardipine), hormonal agents (e.g. calcitonin, estradiol, estron, estriol, polyestradiol, polyestriol, dienestrol, diethylstilbestrol, progesterone, dydrogesterone, cyproterone, danazol, testosterone, calcitonin), contraceptive agents (e.g. ethinyl estradiol, lynestrenol, etynodiol, norethisterone, mestranol, norgestrel, levonorgestrel, desogestrel, medroxyprogesterone), antithrombotic agents (e.g. fragmin, heparin, warfarin), diuretics (e.g. hydrochiorothiazide, flunarizine, minoxidil), antihypertensive agents (e.g. propanolol, metoprolol, cionidine, pindolol), chemical dependency drugs (e.g. nicotine, methadone), local anaesthetics (e.g. lidocaine, prilocaine, benzocaine), corticosteroids (e.g. beclomethasone, betamethasone, clobetasol, desonide, desoxymethasone, dexamethasone, diflucortolone, flumethasone, fluocinolone acetonide, fluocinonide, hydrocortisone, methyiprednisolon, triamcinolone acetonide, budesonide, halcinonide), dermatological agents (e.g. nitrofurantoin, dithranol, clioquinol, hydroxyquinoline, isotretinoin, methoxsalen, methotrexate, tretinoin, trioxsalen, salicylic acid, penicillamine), and the like.

Examples of specific active substances are steroids such as estradiol, progesterone, norethindrone, levonorgestrol, ethynodiol, levenorgestrel, norgestimate, gestanin, desogestrel, 3-keton-desogestrel, demegestone, promethoestrol, testosterone, spironolactone, and esters thereof; a nitro compound such as amyl nitrates, nitroglycerine and isosorbide nitrates; an amine compound such as prilocaine, oxybutyninchloride, lidocaine, benzocaine, nicotine, chlorpheniramine, terfenadine, triprolidine, propanolol and metoprolol; an oxicam derivative such as piroxicam; a mucopolysaccharide such as thiomucase; an opioid such as morphine and morphine-like drugs such as buprenorphine, oxymorphone, hydromorphone, levorphanol, fentanyl and fentanyl derivatives and analogs; a prostaglandin such as a member of the PGA, PGB, PGE and PGF series, such as e.g. misoprostol and enaprostil; a benzamide such as metoclopramide and scopolamine; a peptide such as growth-hormone releasing factors, growth factors (EGF, TGF, PDGF and the like), somatostatin and insulin; a xanthine such as caffeine and theophylline; a catechloamine such as ephedrine, salbutamol and terbutaline; a dihydropyridine such as nifedipine; a thiazide such as hydrochlorothiazide and flunarizine; a sydnonimine such as molsidomine; a sulfated polysaccharide such as heparin; and a phosphodiesterase type 5 (PDE5) inhibitor such as sildenafil, tadalafil or vardenafil.

A preferred composition is the combination of a compound of Formula (I) and an analgesic, preferably hydromorphine hydrochloride or morphine sulphate. An alternative preferred combination is a compound of Formula (I) and nicotinic acid. Yet a further alternative preferred combination is a compound of Formula (I) and an anti-impotence agent, preferably sildenafil, tadalafil or vardenafil. Further preferred compositions include a compound of Formula (I) combined with a corticosteroid, a peptide or a hormone.

The invention will be illustrated by the following non-limiting examples.

*Example 1.* Delmopinol as transdermal penetration enhancer for strong analgesic agents.

**Method.**

The permeation test was carried out using a Franz diffusion cell with a diffusion area of 1.8 $cm^2$. A human full-skin sample was positioned in the cell with a 0.1 M acetate buffer solution (pH 4.5) as receiving phase on the "receptor side of the skin. 180 $\mu$l of 10 % hydromorphone hydrochloride, 10 % morphine sulphate in distilled water, with or without the addition of 25% delmopinol hydrochloride was used on the "donor" side of the skin. Penetration with and without addition of delmopinol hydrochloride was studied in 2 parallel tests (3 cells per test).

2 ml samples from the receiving phase were analyzed over a two week period by a HPLC method which had

been found not to be influenced by delmopinol concentrations below 0.2 mg/ml.

The permeation of hydromorphone increased considerably in the presence of delmopinol as indicated by a steady state flux value of 58 μg/h/cm$^2$ compared to 0.2 μg/h/cm$^2$, without the addition of delmopinol (see Table 1). This means that in the presence of 25 % delmopinol the permeation of hydromorphone hydrochloride is increased with a factor of about 290, which is a very high value compared with other vehicles studied in the literature.

**Table 1.**

| Enhancer | Flux μg/h/cm$^2$ |
|---|---|
| none | 0.2 |
| delmopinol | 58 |

In the morphine sulphate experiments a steady state flux of about 26 μg/h/cm$^2$ was observed. The permeation of this substance without the delmopinol vehicle was however too low to be determined by the analysis method used and the enhancement due to delmopinol could therefore not be quantified.

*Example 2.* Delmopinol analogues as transdermal penetration enhancers for strong analgesic agents.

**Method.**

The permeation test was carried out using a Franz diffusion cell with a diffusion area of 1.8 cm$^2$ as in Example 1. A human full-skin sample was positioned in the cell with a 0.1 M acetate buffer solution (pH 4.5) as receiving phase on the "receptor" side of the skin. 180 μl of 5 % hydromorphone hydrochloride in distilled water with the addition of 25 % delmopinol analogue was used on the "donor" side of the skin. Penetration with or without addition of different delmopinol analogues was studied in 5 parallel tests (3 cells per test). The results are shown in Table 2.

When no enhancing agent was added no detectable amount of hydromorphone hydrochloride was found on the "receptor" side.

**Table 2.**

| Enhancer | Flux μg/h/cm$^2$ |
|---|---|
| M 1523 (octapinol) | 13 |
| M 1676 | 19 |
| M 1713 | 4.8 |

Example 3 Delmopinol as a transdermal penetration enhancer for nicotinic acid.

**Method.**

The permeation test was carried out using a Franz diffusion cell with a diffusion area of 1.8 cm$^2$ as in Example 1. A human full-skin sample was positioned in the cell with a 0.05 M phosphate buffer solution (pH 7.4) as receiving phase on the "receptor" side of the skin. 180 μl of 1 % nicotinic acid in distilled water with and without the addition of 25% delmopinol hydrochloride was used on the "donor" side of the skin. The nicotinic acid was $^{14}$C-labeled. Penetration with and without addition of delmopinol hydrochloride was studied in 2 parallel tests (3 cells per test).

Radioactivity in the receiving" phase was read at different time intervals over a one week period. The steady state flux of nicotinic acid was 0.15 μg/h/cm$^2$ without enhancer while it increased to 1.5 μg/h/cm$^2$ in the presence of 25% delmopinol hydrochloride, as shown in Table 3.

**Table 3.**

| Enhancer | Flux μg/h/cm$^2$ |
|---|---|
| none | 0.15 |
| delmopinol | 1.5 |

*Example 4.* Delmopinol as transdermal penetration enhancer for mannitol

**Method.**

The permeation test was carried out using a Franz diffusion cell with a diffusion area of 1.8 $cm^2$, as in Example 1. A human full-skin sample was positioned in the cell with a 0.05 M phosphate buffer solution (pH 7.4) as receiving phase on the "receptor" side of the skin. 180 μl of 1 % mannitol in distilled water with and without the addition of 1, 5, 10 and 25 % delmopinol hydrochloride respectively was used on the "donor" side of the skin. The mannitol was $^3H$-labeled. Penetration with and without addition of delmopinol hydrochloride was studied in 5 parallel tests (3 cells per test).

Radioactivity in the receiving phase was read at different time intervals over a one week period. A very pronounced effect of the penetration enhancer was seen, but only little if any difference between the concentrations 5, 10 and 25 %, as shown in Table 4.

**Table 4.**

| % delmopinol hydrochloride | Flux μg/h/$cm^2$ |
|---|---|
| 0 | 6.6 |
| 1 | 140 |
| 5 | 230 |
| 10 | 230 |
| 25 | 260 |

*Example 5.* Delmopinol as transdermal penetration enhancer for thyroid-releasing hormone (TRH)

**Method.**

The permeation test was carried out using a Franz diffusion cell with a diffusion area of 1.8 $cm^2$ as in Example 1. A human full-skin sample was positioned in the cell with a 0.05 M phosphate buffer solution (pH 6.0) as receiving phase on the "receptor" side of the skin. 180 μl of 5 % thyroid-releasing hormone (TRH) in dist. water with and without the addition of 25% delmopinol hydrochloride was used on the "donor" side of the skin. The TRH was $^3H$-labeled. Penetration with and without addition of delmopinol hydrochloride was studied in 2 parallel tests (4 cells per test).

Radioactivity in the receiving phase was read at different time intervals over a one week period. This is, to the knowledge of the inventors, a rare example where a penetration enhancer has a pronounced effect on the delivery of a peptide through human skin as can be seen from Table 5, showing steady state fluxes.

**Table 5.**

| Enhancer | Flux ng/h/$cm^2$ |
|---|---|
| none | 4 |
| delmopinol | 34 |

*Example 6.* Effect of octapinol (M1523) on nasal absorption of peptides

**Method.**

Tests on the enhancement effect of octapinol (M1523), i.e.4-(2-propylpentyl)-1- 2-propylpentyl)-1-piperidine-ethanol, a compound according to Formula (I), for nasal absorption of peptides were carried out on Sprague Dawley male rats with an individual weight of 250-300 g. The rats were fasted for 15-17 hours and anaesthetized prior to the tests. Trachea and arteria carotis were catheterized with a polyethylene tube. The peptide used for the tests was insulin.

The rats were divided into 5 groups and were administered through the nostril via a polyethylene tube with

- 1 % octapinol hydrochloride (placebo group)
- insulin (Actrapid ® Human from NovoNordisk, Denmark) 5 IE/kg body weight in physiological NaCl (control group)
- insulin 2.5 IE/kg body weight with 0.02% octapinol hydrochloride (test group)
- insulin 2.5 IE/kg body weight with 0.2% octapinol hydrochloride (test group)
- insulin 2.5 IE/kg body weight with 1% octapinol hydrochloride (test group)

The pH-value was 3.1 in the test solutions.
The administration volume per rat was 25-30 μl.

There were 4 rats in the placebo group, 3 rats in the control group and 6 rats in each of the three test groups.

During the initial tests octapinol hydrochloride was administered nasally together with insulin 5 IE/kg body weight. At an early stage, pronounced reductions in plasma glucose with 1% octapinol hydrochloride were noted.

These reductions were in the order of 85 - 90 % of the initial plasma glucose value. These reductions were so important that a reduction in the insulin dose in the further tests was regarded as necessary. The reason to initially use the higher dose of insulin 5 IE/kg body weight was that octapinol hitherto had not been used for nasal enhancement and thus a suitable dose was not known.

Placebo, 1% octapinol hydrochloride was administered nasally to four rats in order to investigate the effect of pure octapinol hydrochloride on the reduction in plasma glucose. No significant change in plasma glucose level was noted.

In order to ensure that octapinol does not degrade insulin, but that insulin combined with octapinol has a glucose reducing effect, 1% octapinol hydrochloride + 0.2 IE insulin/kg body weight was administered intravenously and the reduction in plasma glucose was determined during 60 minutes. A reduction in plasma glucose of about 40 % was observed after 20 minutes.

The pH-value 3.1 was chosen in order to avoid precipitations when mixing octapinol hydrochloride with insulin. Precipitations occurred at higher pH-values.

In the control and test groups 150 μl blood was taken from arteria carotis 0, 10, 20, 30, 40, 50, 60, 120, 180 and 240 minutes respectively after administration.

The plasma content of glucose was determined through measurement of the amount NADH formed in accordance with the below reactions.

$$\text{glucose} + \text{ATP} \xrightarrow{\text{HK}} \text{glucose-6-phosphate} + \text{ADP}$$

$$\text{glucose-6-phosphate} + \text{NAD}^+ \dashrightarrow \text{6-phosphogluconate} + \text{NADH} + \text{H}^+$$

The change in glucose content was set off against time and the area below the graph (AUC) was calculated with the trapezium method. The results are provided in Figure 1.

The total reduction in plasma glucose (D%) and the maximal reduction in plasma glucose after nasal administration of insulin with octapinol hydrochloride is given in Table 6. The total reduction in plasma glucose (D%) was calculated according to Hirai et al (Int. J. Pharmaceut., 9,173-184: 1981).

$$D\,\% = \frac{AUC_c - AUC_{in}}{AUC_c} \times 100$$

$AUC_c$= area under the graph for intranasally administered insulin 5 IE/kg body weight in physiological Na Cl (control group)

$AUC_{in}$= area under the graph for intranasally administered insulin in octapinol solution

**Table 6**

Total reduction in plasmaglucose (D%) and maximum reduction in plasma glucose after nasal administration of insulin with octapinol hydrochloride. The values are presented as mean values and standard deviation (S.D.)

| Concentration octapinol hydrochloride (%) | Dose of insulin (IE/kg) | D (%) | Maximum reduction in plasma glucose (%) | n (number of rats) |
|---|---|---|---|---|
| control | 5 | - | 4.2 (4.9) | 3 |
| 0.02 | 2.5 | 3.6 (3.2) | 3.5 (2.9) | 6 |
| 0.2 | 2.5 | 9.5 (5.3) | 6.5 (3.5) | 6 |
| 1 | 2.5 | 23.8 (5.8) | 39.1 (11.3) | 6 |

The above results indicate clearly that the substances represented by formula (I) have a great potential as penetration enhancing vehicles to be used in the transdermal and transmucosal administration of physiologically active compounds.

## Claims

**1.** A composition comprising:

(a) 3-(4-propylheptyl)-4-morpholine-ethanol or a pharmaceutically acceptable salt thereof, 4-(2-propylpentyl)-1-piperidine-ethanol or a pharmaceutically acceptable salt thereof, or 4-(2-hydroxyethyl)-4-methyl-3-(4-propylheptyl) morpholinium chloride; and
(b) a pharmacologically active agent selected from the group consisting of an analgesic, nicotinic acid and a peptide.

**2.** A composition according to claim 1 wherein the pharmacologically active agent is an analgesic.

**3.** A composition according to claim 2 wherein the analgesic is buprenorphine, codeine, fentanyl, morphine or hydromorphone.

**4.** A composition according to claim 1 wherein the analgesic is hydromorphone hydrochloride.

**5.** A composition according to claim 1 wherein the analgesic is morphine sulfate.

**6.** A composition according to claim 1 wherein the pharmacologically active agent is nicotinic acid.

**7.** A composition according to claim 1 wherein the pharmacologically active agent is a peptide.

**8.** A composition according to claim 7 wherein the peptide is insulin.

**9.** A composition according to any preceding claim, for nasal administration.

**10.** A composition according to any one of claims 2 to 5 for use in the prevention or treatment of pain.

**11.** A composition according to claim 6 for use in the prevention or treatment of nicotine dependence.

**12.** A composition according to claim 8 for use in reducing plasma glucose.

**13.** Use of a composition comprising 3-(4-propylheptyl)-4-morpholine-ethanol or a pharmaceutically acceptable salt thereof, 4-(2-propylpentyl)-1-piperidine-ethanol or a pharmaceutically acceptable salt thereof, or 4-(2-hydroxyethyl)-4-methyl-3-(4-propylheptyl) morpholinium chloride, as a transdermal or transmucosal drug-delivery vehicle.

**14.** A woven material comprising a composition as defined in any of claims 1 to 8.

**15.** An adhesive patch comprising a woven material according to claim 14.

**16.** A container for nasal administration comprising a composition as defined in any of claims 1 to 8.

**17.** A compound having the formula 4-(2-hydroxyethyl)-4-methyl-3-(4-propylheptyl) morpholinium chloride.

## Patentansprüche

**1.** Eine Zusammensetzung umfassend:

(a) 3-(4-Propylheptyl)-4-morpholin-ethanol oder ein pharmazeutisch verträgliches Salz davon, 4-(2-Propylpentyl)-1-piperidin-ethanol oder ein pharmazeutisch verträgliches Salz davon, oder 4-(2-Hydroxyethyl)-4-methyl-3-(4-propylheptyl) Morpholiniumchlorid;

und
(b) einen pharmakologischen Wirkstoff, ausgewählt aus der Gruppe bestehend aus einem Schmerzmittel, Nicotinsäure und einem Peptid.

**2.** Eine Zusammensetzung gemäß Anspruch 1, wobei der pharmakologische Wirkstoff ein Schmerzmittel ist.

**3.** Eine Zusammensetzung gemäß Anspruch 2, wobei das Schmerzmittel Buprenorphin, Codein, Fentanyl, Morphin oder Hydromorphon ist.

**4.** Eine Zusammensetzung gemäß Anspruch 1, wobei das Schmerzmittel Hydromorphonhydrochlorid ist.

**5.** Eine Zusammensetzung gemäß Anspruch 1, wobei das Schmerzmittel Morphinsulfat ist.

**6.** Eine Zusammensetzung gemäß Anspruch 1, wobei der pharmakologische Wirkstoff Nicotinsäure ist.

**7.** Eine Zusammensetzung gemäß Anspruch 1, wobei der pharmakologische Wirkstoff ein Peptid ist.

**8.** Eine Zusammensetzung gemäß Anspruch 7, wobei das Peptid Insulin ist.

**9.** Eine Zusammensetzung gemäß einem der vorherigen Ansprüche, zur nasalen Verabreichung.

**10.** Eine Zusammensetzung gemäß einem der Ansprüche 2 bis 5 zur Verwendung in der Vorbeugung oder Behandlung von Schmerz.

**11.** Eine Zusammensetzung gemäß Anspruch 6 zur Verwendung in der Vorbeugung oder Behandlung von Nikotinabhängigkeit.

**12.** Eine Zusammensetzung gemäß Anspruch 8 zur Verwendung in der Plasmaglukoseverringerung.

**13.** Verwendung einer Zusammensetzung umfassend 3-(4-Propylheptyl)-4-morpholin-ethanol oder ein pharmazeutisch verträgliches Salz davon, 4-(2-Propylpentyl)-1-piperidin-ethanol oder ein pharmazeutisch verträgliches Salz davon, oder 4-(2-Hydroxyethyl)-4-methyl-3-(4-propylheptyl) Morpholiniumchlorid, als ein transdermales oder transmukosales Vehikel zur Arzneimittelabgabe.

**14.** Ein gewobenes Material umfassend eine Zusammensetzung wie definiert in einem der Ansprüche 1 bis 8.

**15.** Ein adhäsives Pflaster umfassend ein gewobenes Material gemäß Anspruch 14.

**16.** Ein Behältnis zur nasalen Verabreichung umfassend eine Zusammensetzung wie in einem der Ansprüche 1 bis 8 definiert.

**17.** Eine Verbindung der Formel 4-(2-Hydroxyethyl)-4-methyl-3-(4-propylheptyl) Morpholiniumchlorid.

## Revendications

**1.** Composition comprenant:

(a) du 3-(4-propylheptyl)-4-morpholine-éthanol ou un sel pharmaceutiquement acceptable de celui-ci, du 4-(2-propylpentyl)-1-pipéridine-éthanol ou un sel pharmaceutiquement acceptable de celui-ci ou du chlorure de 4-(2-hydroxyéthyl)-4-méthyl-3-(4-propylheptyl)morpholinium ;
et
(b) un agent pharmacologiquement actif choisi dans le groupe consistant en un analgésique, l'acide nicotinique et un peptide.

**2.** Composition selon la revendication 1 où l'agent pharmacologiquement actif est un analgésique.

**3.** Composition selon la revendication 2 où l'analgésique est la buprénorphine, la codéine, le fentanyl, la morphine ou

l'hydromorphone.

4. Composition selon la revendication 1 où l'analgésique est le chlorhydrate d'hydromorphone.

5. Composition selon la revendication 1 où l'analgésique est le sulfate de morphine.

6. Composition selon la revendication 1 où l'agent pharmacologiquement actif est l'acide nicotinique.

7. Composition selon la revendication 1 où l'agent pharmacologiquement actif est un peptide.

8. Composition selon la revendication 7 où le peptide est l'insuline.

9. Composition selon l'une quelconque des revendications précédentes, pour l'administration nasale.

10. Composition selon l'une quelconque des revendications 2 à 5 destinée à être utilisée dans la prévention ou le traitement de la douleur.

11. Composition selon la revendication 6 destinée à être utilisée dans la prévention ou le traitement de la dépendance à la nicotine.

12. Composition selon la revendication 8 destinée à être utilisée dans la réduction du glucose plasmatique.

13. Utilisation d'une composition comprenant du 3-(4-propylheptyl)-4-morpholine-éthanol ou un sel pharmaceutiquement acceptable de celui-ci, du 4-(2-propylpentyl)-1-pipéridine-éthanol ou un sel pharmaceutiquement acceptable de celui-ci ou du chlorure de 4-(2-hydroxyéthyl)-4-méthyl-3-(4-propylheptyl)morpholinium, comme véhicule de d'apport de médicament transdermique ou transmuqueux.

14. Matière tissée comprenant une composition telle que définie dans l'une quelconque des revendications 1 à 8.

15. Timbre adhésif comprenant une matière tissée selon la revendication 14.

16. Récipient pour l'administration nasale comprenant une composition telle que définie dans l'une quelconque des revendications 1 à 8.

17. Composé ayant la formule chlorure de 4-(2-hydroxyéthyl)-4-méthyl-3-(4-propylheptyl)morpholinium.

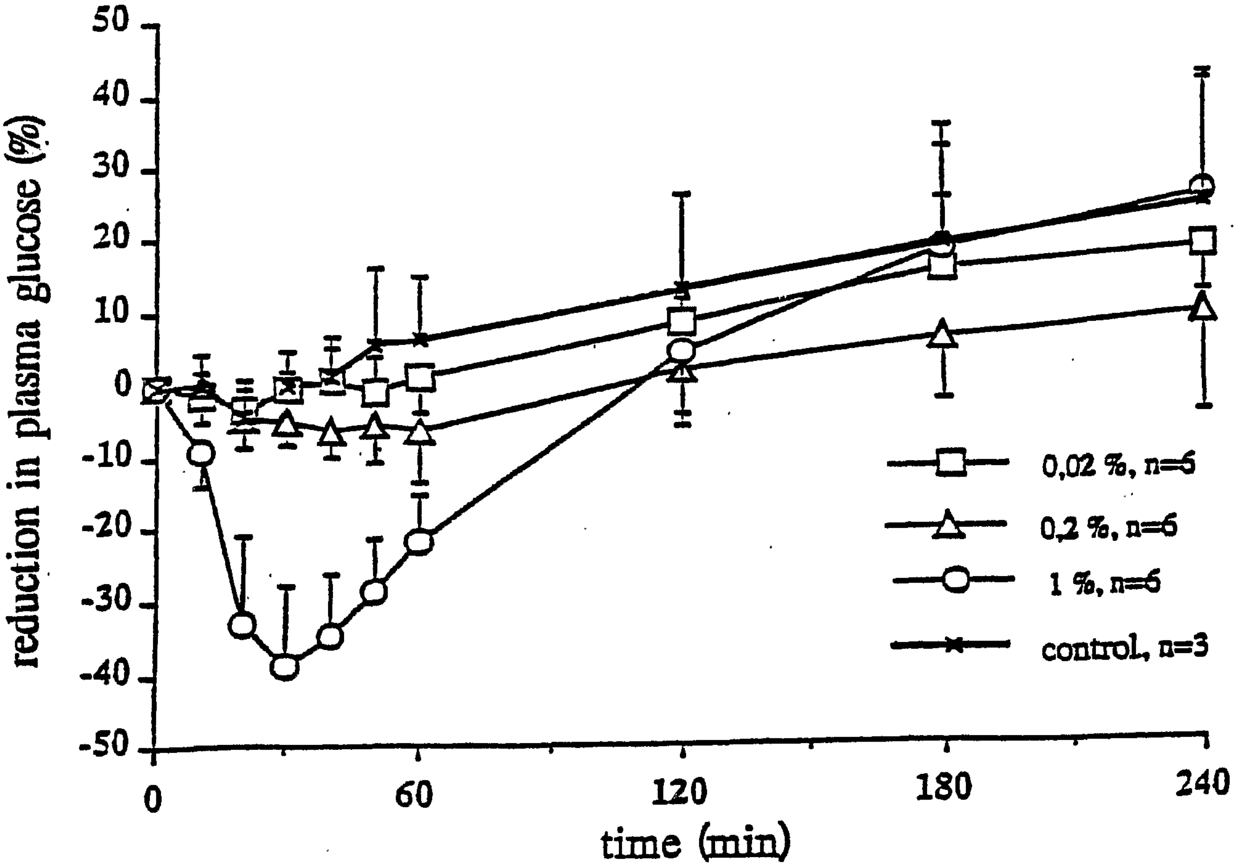
reduction in plasma glucose (%)
50
40
30
20
10
0
-10
-20
-30
-40
-50
0
60
120
180
240
time (min)
0,02 %, n=6
0,2 %, n=6
1 %, n=6
control, n=3

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4235875 A
- US 4894221 A
- US 4636382 A
- WO 9014342 A


### Non-patent literature cited in the description


- Penetration enhancers and their use in Transdermal Therapeutic Systems. **KENNETH A. WALTERS ; HADGRAFT ; GUY.** Transdermal Drug Delivery. Marcel Dekker Inc, 1989, 197-229
- *CHEMICAL ABSTRACTS,* 98092-92-3
- **HIRAI et al.** *Int. J. Pharmaceut.,* 1981, vol. 9, 173-184